# EUROPEAN PATENT SPECIFICATION

(11) **EP 4 225 184 B1**
(45) Date of publication and mention of the grant of the patent: **25.03.2026**
(21) Application number: 21805703.2
(22) Date of filing: 05.10.2021
(51) Int. Cl.: A61B 18/14

(54) **ARTICULATING KNIFE ACTUATOR FOR ELECTROSURGICAL INSTRUMENT**
BEWEGLICHER MESSERAKTUATOR FÜR EIN ELEKTROCHIRURGISCHES INSTRUMENT
ACTIONNEUR DE COUTEAU ARTICULÉ POUR INSTRUMENT D'ÉLECTROCHIRURGIE

(30) Priority: 05.10.2020 US 202063087530 P
(43) Date of publication of application: 16.08.2023
(73) Proprietor: CONMED Corporation, Largo, FL 33773 (US)
(72) Inventor: SZABO, Aaron, Swanton, OH 43558 (US); WILLIAMS, Mason, Centennial, CO 80122 (US)
(74) Representative: Strehl & Partner mbB
(86) International application number: PCT/US2021/053546
(87) International publication number: WO 2022/076392

(56) References cited:
- US-A1- 2017 181 789
- US-A1- 2019 008 537

## Description

### BACKGROUND OF THE INVENTION

### 1. FIELD OF THE INVENTION

The present invention is defined in the appended claims 1 and 10.

### 2. DESCRIPTION OF THE RELATED ART

Electrosurgical vessel sealers are surgical instruments that are used for the occlusion of blood vessels and halting of bleeding during surgical procedures. The electrodes of the vessel sealer are carried by a pair of opposing jaws mounted to the end of an elongated shaft and interconnected to an electrosurgical generator that can selectively supply radiofrequency (RF) energy to the electrodes. A user may close the jaws around a vessel to be sealed by squeezing a lever associated with a handle assembly. The vessel may then be sealed by supplying the RF energy to the clamped vessel and the vessel can be severed by extending a knife blade along tracks formed in the jaws. Conventional approaches to driving the knife blade do not allow for continuous rotation of the shaft. Accordingly, there is a need for an approach that can allow the shaft of the instrument to rotation through 360 degrees while still allowing a user to operate the knife blade once the shaft is positioned as desired. US 2017/0181789 A1 discloses a surgical instrument in line with the preamble of claim 1.

### BRIEF SUMMARY OF THE INVENTION

The present invention is a knife driving assembly that can advance and retract a knife along a knife blade pathway defined by the jaws of the surgical instrument regardless of continuous rotation of the shaft and jaws. In a first embodiment, a surgical instrument according to the present invention comprises a housing having a shaft extending along a longitudinal axis and supporting to a pair of jaws that define a knife pathway and a lever pivotally mounted to housing and extending into the housing. A knife actuator is coupled to the lever at a first end and extending longitudinally within housing to a second end. A bearing sleeve is coupled to the second end of the knife actuator. A drive shaft is secured to the bearing sleeve for moving a knife along the knife pathway in response to movement of the bearing sleeve. The lever may include a bearing cup mechanically coupling the lever to the knife actuator. The second end of the knife actuator may comprise a fork extending about the bearing sleeve. The bearing sleeve may include a pair of bearing members positioned on either side of the fork of the knife actuator. The shaft and the drive shaft may be rotatable through 360 degrees. Pivotal movement of the level between a first position and a second position causes the knife actuator to drive the bearing sleeve from a retracted position to an extended position. The bearing sleeve may biased toward the first position to retract the knife blade after use.

In another embodiment, the present invention is a method of making a surgical device having a knife blade that can retract and extend within jaws that are free to continuously rotate. The method includes providing a lever pivotally mounted to a housing and extending partially therein. The lever is coupled to a first end of a knife actuator that extends longitudinally within the housing. A second end of the knife actuator is coupled to a bearing sleeve that extends around a rotatable shaft of the surgical device. The knife blade is fixed to the bearing sleeve so that movement of the lever from a first position to a second position advances the knife actuator distally within the housing and drives the bearing sleeve along the shaft to move the knife blade from a retracted position to an extended position within the jaws.

### BRIEF DESCRIPTION OF THE SEVERAL VIEWS OF THE DRAWING(S)

The present invention will be more fully understood and appreciated by reading the following Detailed Description in conjunction with the accompanying drawings, in which:
FIG. 1 is a perspective view of an electrosurgical system having an electrosurgical instrument with a knife actuating assembly according to the present invention;
FIG. 2 is a perspective view of the jaws of electrosurgical instrument that can support a knife driven by a knife actuating assembly according to the present invention;
FIG. 3 is schematic of the lower jaw showing movement of the knife blade from the retracted position to the extending position in response to movement of the knife actuating assembly according to the present invention;
FIG. 4 is a perspective view of a knife actuating assembly according to the present invention;
FIG. 5 is a schematic showing the movement of the knife actuating assembly according to the present invention from a first position to a second position in response to manual movement of the lever;
FIG. 6 is a perspective view of the lever and knife actuator according to the present invention; and
FIG. 7 is a schematic of the coupling of the lever and the knife actuator according to the present invention.

### DETAILED DESCRIPTION OF THE INVENTION

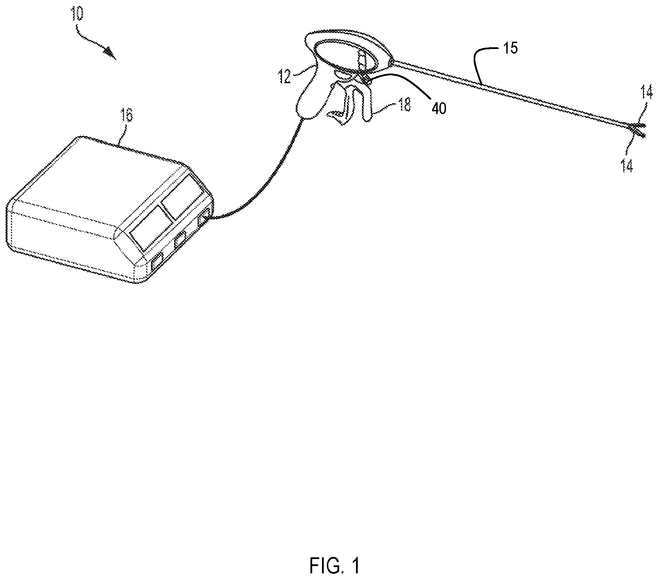

Referring to the figures, wherein like numeral refer to like parts throughout, there is seen in FIG. 1 an electrosurgical system 10 comprising a vessel sealer 12 having a pair of conductive opposing jaws 14 that are interconnected to an electrosurgical generator 16 that can supply RF energy to electrodes of jaws 14 for the desiccation of a blood vessel trapped between jaws 14. As is known in the art, jaws 14 are pivotally mounted to a longitudinally extending shaft 15 of vessel sealer 12 for movement between an open position and a closed position in response to a user operating a lever 18 extending from vessel sealer 12.

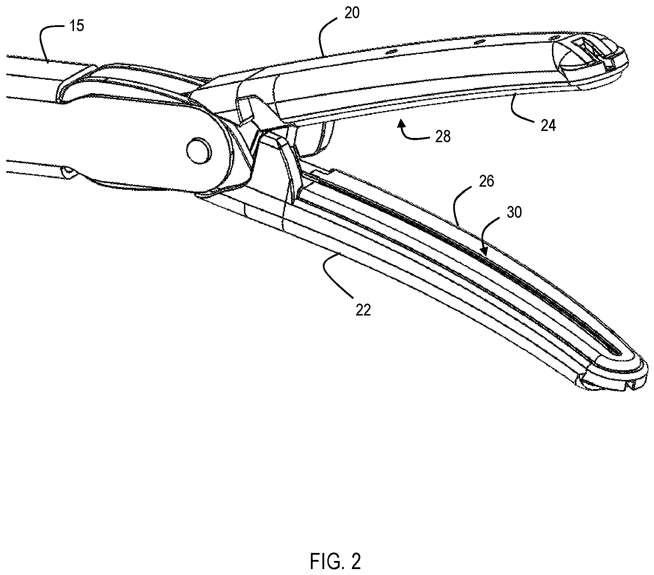

Referring to FIG. 2, jaws 14 are comprised of two opposing jaw members 20 and 22. Jaw members 20 and 22 support inner conductive faces 24 and 26 that are electrically isolated from the rest of jaws 14 and sealer 12 and interconnected to electrosurgical generator 16. Inner conductive faces 24 and 26 have tracks 28 and 30 formed therethrough, respectively, so that when jaw members 20 and 22 are closed, tracks 28 and 30 align to define a knife pathway that intersects the region between jaw members 20 and 22 where tissue is desiccated between jaws 14 when they are energized.

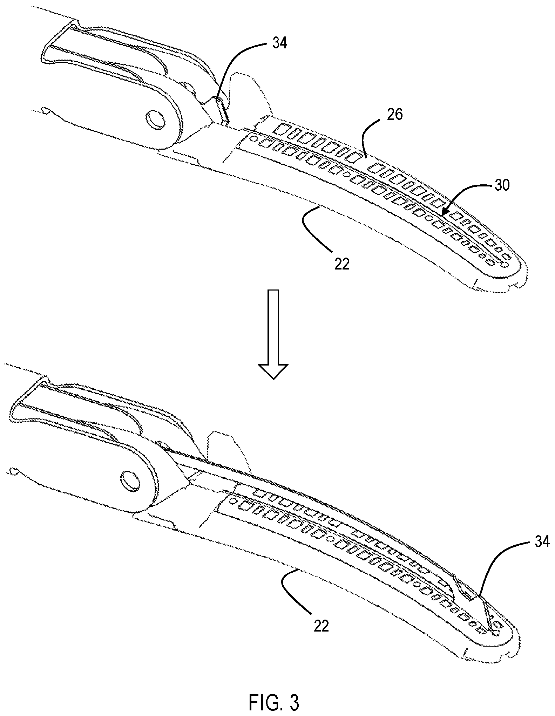

Referring to FIG. 3, a knife blade 34 is aligned with and positioned in the knife pathway defined by tracks 28 and 30 and may be selectively extended through and withdrawn from the knife pathway, such as by a user operating a lever 40 pivotally mounted to the housing 42 of sealer 12, to sever any tissue trapped in jaws 14. As described herein, knife blade 34 may be moved between the retracted position and the extended position regardless of the orientation of the jaws. For example, a user of vessel sealer 12 may rotate shaft 15 through 360 degrees to position jaws 14 is a particular orientation for a procedure without compromising the ability of knife blade 34 to be driven into the extended position to sever any tissue captured in jaws 14.

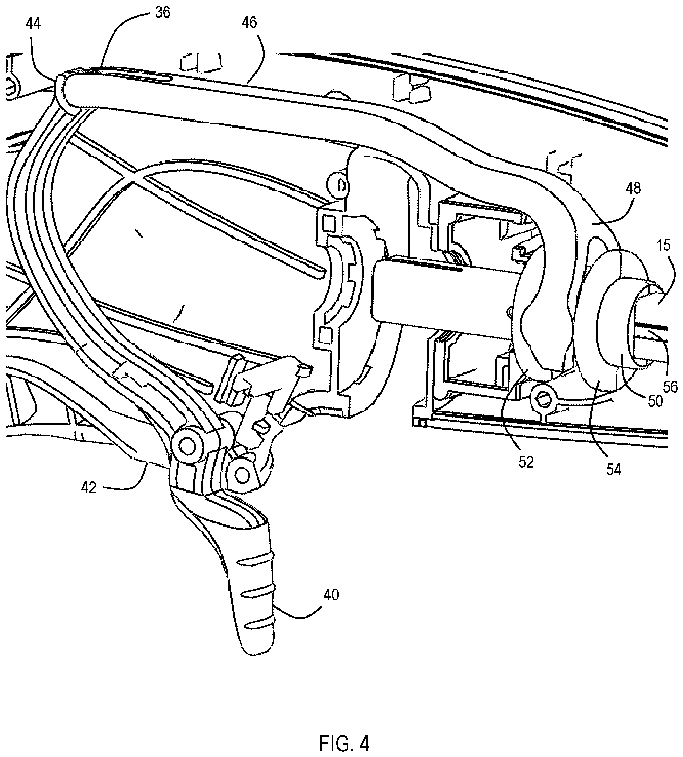

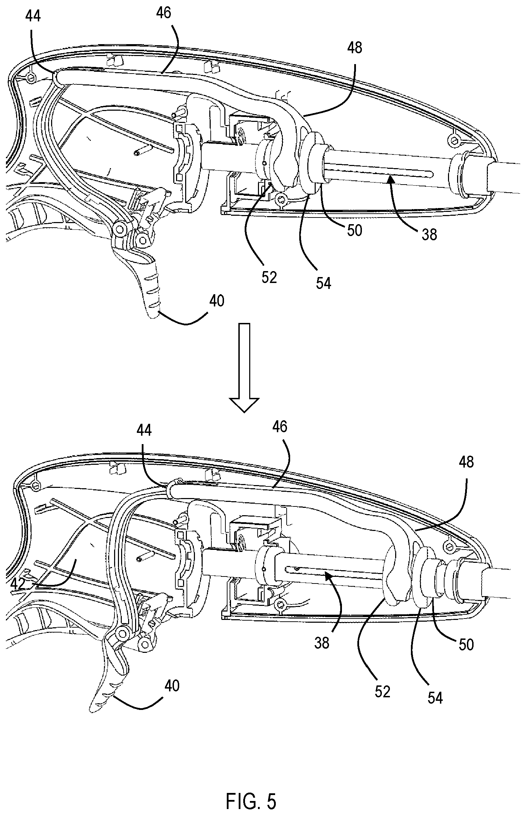

Referring to FIG. 4, a lever 40 is pivotally mounted to the housing 42 and extends into housing 42 to terminate in a bearing cup 44. Bearing cup 44 providing a mechanical connection to a first end 36 of a knife actuator 46 that engages cup 44 and extends longitudinally along the inside of housing 42 to a second end forming a two-armed fork 48 that extends around and is mechanically coupled to a bearing sleeve 50. Bearing sleeve 50 includes opposing bearing members 52 and 54 that are positioned longitudinally about fork 48 so that movement of knife actuator 46 can drive bearing sleeve 50 longitudinally. Bearing sleeve 50 is secured to shaft 15 for rotation therewith. For example, bearing sleeve 50 may have an internal geometry keyed to the outer geometry of shaft 15. Bearing sleeve 50 is secured to a drive shaft 56 that is positioned within and extends inside shaft 15 of vessel sealer 12 and is longitudinally moveable relative thereto. For example, as seen in FIG. 5, shaft 15 includes longitudinal slots 38 permitting bearing sleeve 50 to be secured to drive shaft 56, while allowing bearing sleeve 50 to translate longitudinally along shaft 15. Drive shaft 56 is fixed to knife blade 34 so that longitudinal movement of bearing sleeve 50 along shaft 15 will push drive shaft 56 longitudinally and thus move knife 34 along the knife pathway of jaws 14. Shaft 15 is mounted to housing 42 for continuous rotation about its longitudinal axis and jaws 14 are fixed relative to shaft, thereby allowing a user to position jaws 14 as desired by rotating shaft 15. Bearing sleeve 50 is capable of moving drive shaft 56 longitudinally regardless of the rotational positioning of bearing sleeve 50 along with shaft 15.

Referring to FIG. 5, pivotal movement of the exposed end of lever 40 proximally toward housing 42 will drive knife actuator 46 so that knife actuator 46 pushes bearing sleeve 50 distally, thereby causing shaft 56 to drive a knife blade coupled to shaft longitudinally along the knife pathway of jaws 14 to sever any tissue trapped therein. Lever 40 is thus moveable from a first position to a second position to cause knife actuator 46 to drive bearing sleeve 50 distally along shaft 15, which will move the knife blade from a retracted position to an extended position to sever tissue trapped in jaws 14. As is known in the art, bearing sleeve 50 may be biased to return to the retracted position in the absence of a user force being applied to lever 40, thereby withdrawing knife from the extended position in the knife pathway to the retracted position. Alternatively, a user could manually move lever 40 back to the first position to retract knife 34 from knife pathway.

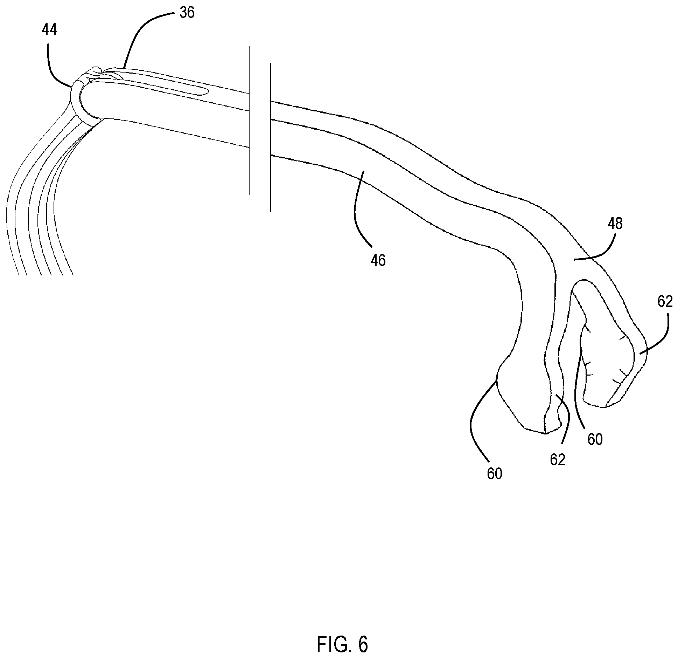

Referring to FIG. 6, each arm of fork 48 has bearing surfaces 60 and 62 that extend proximately and distally to engage bearing members 52 and 54, respectively. Bearing surfaces 60 and 62 allow for the transmission of forces to and from bearing sleeve 50 regardless of rotational positioning of sleeve 50. Bearing surfaces 60 and 62 also for the transmission of forces to and from bearing sleeve 50 when knife actuator 46 is driven by lever 40 as the coupling between lever 40 and knife actuator 46 may produce some pivoting of knife actuator 46 due to the mechanical coupling to bearing cup 44. The combination of lever 40, knife actuator 46, and bearing sleeve 50 allows pivoting of lever 40 translate into longitudinal motion of knife actuator 46 and thus bearing sleeve. In addition, the coupling between fork 48 and bearing sleeve 50 allows the longitudinal movement of knife actuator 46 to drive bearing sleeve 50 to actuate the knife while simultaneously allowing shaft 15 to be rotated through 360 degrees without any loss in the ability to operate the knife.

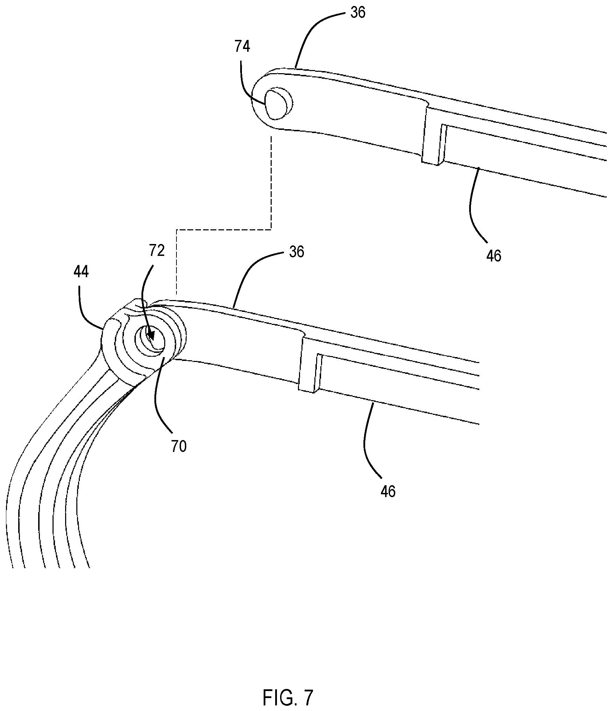

Referring to FIG. 7, bearing cup 44 supports a tab 70 having an aperture 72 formed therethrough. First end 36 of knife actuator 46 is split and includes a post 74 extending transversely to the longitudinal axis of knife actuator 46 to engage aperture 72. Tab 70 of cup 44 and post 74 of knife actuator 46 form a rotational joint that allows pivoting of lever 40 to drive knife actuator 46 longitudinally within housing 42 of vessel sealer 12 and thus operate knife 34.

## Claims

1. A surgical instrument, comprising:
a housing (42) having a shaft (15) extending along a longitudinal axis and supporting to a pair of jaws (14) that define a knife pathway;
a lever (40) pivotally mounted to and extending into the housing (42);
a knife actuator (46) coupled to the lever (40) at a first end (36);
a bearing sleeve (50); and
a knife positioned in the knife pathway and coupled to the bearing sleeve (50) for movement therewith in response to movement of the lever (40) and knife actuator (46),
**characterized in that** the knife actuator (46) extends longitudinally within the housing (42) to a second end, and the bearing sleeve (50) is coupled to the second end of the knife actuator (46).

2. The surgical instrument of claim 1, wherein the lever (40) includes a bearing cup (44) mechanically coupling the lever (40) to the knife actuator (46).

3. The surgical instrument of claim 2, wherein the second end of the knife actuator (46) comprises a fork (48) extending about the bearing sleeve (50).

4. The surgical instrument of claim 3, wherein the bearing sleeve (50) includes a pair of bearing members (52, 54) positioned on either side of the fork (48) of the knife actuator (46).

5. The surgical instrument of claim 4, wherein the shaft (15) is rotatable through 360 degrees.

6. The surgical instrument of claim 5, wherein pivotal movement of the lever (40) between a first position and a second position will cause the knife actuator (46) to drive the bearing sleeve (50) from a retracted position to an extended position.

7. The surgical instrument of claim 6, wherein the bearing sleeve (50) is biased toward the first position.

8. The surgical instrument of claim 7, wherein the bearing cup (44) includes a tab (70) and an aperture (72) formed through the tab (70).

9. The surgical instrument of claim 8, wherein the first end (36) of the knife actuator (46) includes a post (74) engaged with the aperture (72) of the tab (70) of the bearing cup (44).

10. A method of making a surgical device having a knife blade (34) that can retract and extend within jaws (14) that are free to continuously rotate, comprising:
providing a lever (40) pivotally mounted to a housing (42) and extending partially therein;
coupling the lever (40) to a first end (36) of a knife actuator (46) that extends longitudinally within the housing (42);
coupling a second end of the knife actuator (46) to a bearing sleeve (50) that extends around a rotatable shaft (15) of the surgical device; and
fixing the knife blade (34) to the bearing sleeve (50) so that movement of the lever (40) from a first position to a second position advances the knife actuator (46) distally within the housing (42) and drives the bearing sleeve (50) along the shaft (15) to move the knife blade (34) from a retracted position to an extended position within the jaws (14).

11. The method of claim 10, wherein the lever (40) includes a bearing cup (44) that is used to mechanically couple the lever (40) to the knife actuator (46).

12. The method of claim 11, wherein the second end of the knife actuator (46) is coupled to the bearing sleeve (50) by a fork (48) that extends about the bearing sleeve (50).

13. The method of claim 12, wherein the bearing sleeve (50) includes a pair of bearing members (52, 54) positioned on either side of the fork (48) of the knife actuator (46).

## Patentansprüche

1. Chirurgisches Instrument, umfassend:
ein Gehäuse (42) mit einem Schaft (15), der sich entlang einer Längsachse erstreckt und ein Paar Backen (14) stützt, die eine Messerbahn definieren;
einen Hebel (40), der schwenkbar an dem Gehäuse (42) gelagert ist und sich in das Gehäuse (42) hinein erstreckt;
einen Messerantrieb (46), der an einem ersten Ende (36) mit dem Hebel (40) gekoppelt ist;
eine Lagerhülse (50); und
ein Messer, das in der Messerbahn angeordnet und mit der Lagerhülse (50) für eine Bewegung damit als Reaktion auf eine Bewegung des Hebels (40) und des Messerantriebs (46) gekoppelt ist,
**dadurch gekennzeichnet, dass** der Messerantrieb (46) sich längs innerhalb des Gehäuses (42) zu einem zweiten Ende erstreckt und die Lagerhülse (50) mit dem zweiten Ende des Messerantriebs (46) gekoppelt ist.

2. Chirurgisches Instrument nach Anspruch 1, wobei der Hebel (40) eine Lagerschale (44) umfasst, die den Hebel (40) mechanisch mit dem Messerantrieb (46) koppelt.

3. Chirurgisches Instrument nach Anspruch 2, wobei das zweite Ende des Messerantriebs (46) eine sich um die Lagerhülse (50) erstreckende Gabel (48) umfasst.

4. Chirurgisches Instrument nach Anspruch 3, wobei die Lagerhülse (50) ein Paar Lagerelemente (52, 54) umfasst, die auf beiden Seiten der Gabel (48) des Messerantriebs (46) angeordnet sind.

5. Chirurgisches Instrument nach Anspruch 4, wobei der Schaft (15) um 360 Grad drehbar ist.

6. Chirurgisches Instrument nach Anspruch 5, wobei eine Schwenkbewegung des Hebels (40) zwischen einer ersten Stellung und einer zweiten Stellung bewirkt, dass der Messerantrieb (46) die Lagerhülse (50) von einer eingezogenen Stellung zu einer ausgefahrenen Stellung antreibt.

7. Chirurgisches Instrument nach Anspruch 6, wobei die Lagerhülse (50) in Richtung der ersten Stellung vorgespannt ist.

8. Chirurgisches Instrument nach Anspruch 7, wobei die Lagerschale (44) eine Lasche (70) und eine durch die Lasche (70) gebildete Öffnung (72) umfasst.

9. Chirurgisches Instrument nach Anspruch 8, wobei das erste Ende (36) des Messerantriebs (46) einen mit der Öffnung (72) der Lasche (70) der Lagerschale (44) in Eingriff stehenden Zapfen (74) umfasst.

10. Verfahren zum Herstellen einer chirurgischen Vorrichtung mit einer Messerklinge (34), die sich innerhalb von Backen (14) einziehen und ausfahren lässt, die frei sind, kontinuierlich zu rotieren, umfassend:
Bereitstellen eines Hebels (40), der schwenkbar an einem Gehäuse (42) gelagert ist und sich teilweise darin erstreckt;
Koppeln des Hebels (40) mit einem ersten Ende (36) eines Messerantriebs (46), der sich längs innerhalb des Gehäuses (42) erstreckt;
Koppeln eines zweiten Endes des Messerantriebs (46) mit einer Lagerhülse (50), die sich um einen drehbaren Schaft (15) der chirurgischen Vorrichtung erstreckt; und
Befestigen der Messerklinge (34) an der Lagerhülse (50), so dass eine Bewegung des Hebels (40) von einer ersten Stellung zu einer zweiten Stellung den Messerantrieb (46) distal innerhalb des Gehäuses (42) vorschiebt und die Lagerhülse (50) entlang des Schaftes (15) antreibt, um die Messerklinge (34) von einer eingezogenen Stellung zu einer ausgefahrenen Stellung innerhalb der Backen (14) zu bewegen.

11. Verfahren nach Anspruch 10, wobei der Hebel (40) eine Lagerschale (44) umfasst, die verwendet wird, um den Hebel (40) mechanisch mit dem Messerantrieb (46) zu koppeln.

12. Verfahren nach Anspruch 11, wobei das zweite Ende des Messerantriebs (46) mit der Lagerhülse (50) durch eine Gabel (48) gekoppelt ist, die sich um die Lagerhülse (50) erstreckt.

13. Verfahren nach Anspruch 12, wobei die Lagerhülse (50) ein Paar Lagerelemente (52, 54) umfasst, die auf beiden Seiten der Gabel (48) des Messerantriebs (46) angeordnet sind.

## Revendications

1. Instrument chirurgical, comprenant :
un boîtier (42) ayant un arbre (15) s'étendant le long d'un axe longitudinal et supportant une paire de mâchoires (14) qui définissent un chemin de passage de couteau ;
un levier (40) monté pivotant sur et s'étendant jusque dans le boîtier (42) ;
un actionneur (46) de couteau couplé au levier (40) à une première extrémité (36) ;
un manchon (50) de palier ; et
un couteau positionné dans le chemin de passage de couteau et couplé au manchon (50) de palier pour un mouvement avec celui-ci en réponse à un mouvement du levier (40) et de l'actionneur (46) de couteau,
**caractérisé en ce que** l'actionneur (46) de couteau s'étend longitudinalement à l'intérieur du boîtier (42) jusqu'à une deuxième extrémité, et le manchon (50) de palier est couplé à la deuxième extrémité de l'actionneur (46) de couteau.

2. Instrument chirurgical selon la revendication 1, dans lequel le levier (40) inclut une cuvette (44) de palier couplant mécaniquement le levier (40) à l'actionneur (46) de couteau.

3. Instrument chirurgical selon la revendication 2, dans lequel la deuxième extrémité de l'actionneur (46) de couteau comprend une fourchette (48) s'étendant autour du manchon (50) de palier.

4. Instrument chirurgical selon la revendication 3, dans lequel le manchon (50) de palier inclut une paire d'éléments (52, 54) de palier positionnés sur chaque côté de la fourchette (48) de l'actionneur (46) de couteau.

5. Instrument chirurgical selon la revendication 4, dans lequel l'arbre (15) est rotatif sur 360 degrés.

6. Instrument chirurgical selon la revendication 5, dans lequel un mouvement de pivot du levier (40) entre une première position et une deuxième position amènera l'actionneur (46) de couteau à entraîner le manchon (50) de palier d'une position rétractée à une position étendue.

7. Instrument chirurgical selon la revendication 6, dans lequel le manchon (50) de palier est poussé vers la première position.

8. Instrument chirurgical selon la revendication 7, dans lequel la cuvette (44) de palier inclut une languette (70) et une ouverture (72) formée à travers la languette (70).

9. Instrument chirurgical selon la revendication 8, dans lequel la première extrémité (36) de l'actionneur (46) de couteau inclut un montant (74) engagé avec l'ouverture (72) de la languette (70) de la cuvette (44) de palier.

10. Procédé de fabrication d'un dispositif chirurgical ayant une lame (34) de couteau qui peut se rétracter et s'étendre à l'intérieur de mâchoires (14) qui sont libres de tourner de façon continue, comprenant :
la prévision d'un levier (40) monté pivotant sur un boîtier (42) et s'étendant partiellement dans celui-ci ;
l'accouplement du levier (40) à une première extrémité (36) d'un actionneur (46) de couteau qui s'étend longitudinalement à l'intérieur du boîtier (42) ;
l'accouplement d'une deuxième extrémité de l'actionneur (46) de couteau à un manchon (50) de palier qui s'étend autour d'un arbre rotatif (15) du dispositif chirurgical ; et
la fixation de la lame (34) de couteau au manchon (50) de palier de telle sorte qu'un mouvement du levier (40) d'une première position à une deuxième position fait avancer l'actionneur (46) de couteau distalement à l'intérieur du boîtier (42) et entraîne le manchon (50) de palier le long de l'arbre (15) pour déplacer la lame (34) de couteau d'une position rétractée à une position étendue à l'intérieur des mâchoires (14).

11. Procédé selon la revendication 10, dans lequel le levier (40) inclut une cuvette (44) de palier qui est utilisée pour coupler mécaniquement le levier (40) à l'actionneur (46) de couteau.

12. Procédé selon la revendication 11, dans lequel la deuxième extrémité de l'actionneur (46) de couteau est couplée au manchon (50) de palier par une fourchette (48) qui s'étend autour du manchon (50) de palier.

13. Procédé selon la revendication 12, dans lequel le manchon (50) de palier inclut une paire d'éléments (52, 54) de palier positionnés sur chaque côté de la fourchette (48) de l'actionneur (46) de couteau.
